(19)

Europäisches Patentamt

European Patent Office

Office européen des brevets

(11) **EP 4 752 243 A1**

(12) **EUROPEAN PATENT APPLICATION**
published in accordance with Art. 153(4) EPC

(43) Date of publication:
03.06.2026 Bulletin 2026/23

(21) Application number: 24940893.1

(22) Date of filing: 30.09.2024

(51) International Patent Classification (IPC):
**C22B 23/00** $^{(2006.01)}$

(86) International application number:
**PCT/CN2024/122777**

(87) International publication number:
**WO 2026/065408 (02.04.2026 Gazette 2026/14)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(71) Applicants:
• **Gem Co., Ltd.**
**Shenzhen, Guangdong 518101 (CN)**
• **PT ESG New Energy Material**
**Jakarta Selatan, Provinsi DKI Jakarta 12950 (ID)**
• **PT Green Eco Nickel**
**Jakarta Selatan, Provinsi DKI Jakarta, 12950 (ID)**
• **PT QMB New Energy Materials**
**Jakarta Selatan, Provinsi DKI Jakarta, 12950 (ID)**

(72) Inventors:
• **XU, Kaihua**
**Shenzhen, Guangdong 518100 (CN)**
• **BRODJONEGORO, Satryo Soemantri**
**Kota Adm. Jakarta Selatan,**
**Provinsi DKI Jakarta 12950 (ID)**
• **WAN, Wenjing**
**Kota Adm. Jakarta Selatan,**
**Provinsi DKI Jakarta 12950 (ID)**
• **WANALDI, Rizky**
**Kota Adm. Jakarta Selatan,**
**Provinsi DKI Jakarta 12950 (ID)**

• **YANG, Jian**
**Kota Adm. Jakarta Selatan,**
**Provinsi DKI Jakarta 12950 (ID)**
• **PENG, Yaguang**
**Kota Adm. Jakarta Selatan,**
**Provinsi DKI Jakarta 12950 (ID)**
• **HASIBUAN, Andi Syaputra**
**Kota Adm. Jakarta Selatan,**
**Provinsi DKI Jakarta 12950 (ID)**
• **RAHMADI, Piyan**
**Kota Adm. Jakarta Selatan,**
**Provinsi DKI Jakarta 12950 (ID)**
• **KRISTIYANTO, Evan Wahyu**
**Kota Adm. Jakarta Selatan,**
**Provinsi DKI Jakarta 12950 (ID)**
• **KEVIN, Gerardus**
**Kota Adm. Jakarta Selatan,**
**Provinsi DKI Jakarta 12950 (ID)**
• **ISAROYATI, Luluk**
**Kota Adm. Jakarta Selatan,**
**Provinsi DKI Jakarta 12950 (ID)**
• **ARINDA, Shella**
**Kota Adm. Jakarta Selatan,**
**Provinsi DKI Jakarta 12950 (ID)**

(74) Representative: **Biallo, Dario et al**
**Barzanò & Zanardo S.p.A.**
**Via Borgonuovo, 10**
**20121 Milano (IT)**

(54) **METHOD FOR PREPARING NICKEL-COBALT-MANGANESE RAW MATERIAL WITH ION CO-BALANCE IN HYDROMETALLURGY OF LATERITE-NICKEL ORE**

(57) Disclosed is a preparation method for a nickel-cobalt-manganese raw material with ion co-equilibrium in hydrometallurgy of laterite nickel ore, including the following steps: acquiring parameters of an initial material liquid, an actual pH of a material liquid at a reaction endpoint, and a target precipitation rate of a target metal; establishing a first theoretical relationship model between a theoretical pH of the material liquid at the reaction endpoint and the target precipitation rate of the target metal, and obtaining the theoretical pH of the material liquid at the reaction endpoint based on the parameters of the initial material liquid and the first theoretical relationship model; and judging whether to terminate the reaction based on the theoretical pH and the actual pH of the material liquid at the reaction endpoint. The present application guides the MHP precipitation process from the perspective of ionic balance. Through the method of the present application, the theoretical pH of the material liquid at the reaction endpoint can be directly obtained

**EP 4 752 243 A1**

based on the parameters of the initial material liquid and the target precipitation rate of the target metal, thus judging whether to terminate the reaction through the theoretical pH of the material liquid at the reaction endpoint and the actual pH of the material liquid at the reaction endpoint.

S1 — Acquiring the parameters of the initial material liquid, the actual pH of the material liquid at the reaction endpoint, and the target precipitation rate of the target metal

S2 — Constructing a first theoretical relationship model between the theoretical pH of the material liquid at the reaction endpoint and the target precipitation rate of the target metal, and acquiring the theoretical pH of the material liquid at the reaction endpoint based on the parameters of the initial material liquid and the first theoretical relationship model

S3 — Judging whether to stop the reaction based on the theoretical pH of the material liquid at the reaction endpoint and the actual pH of the material liquid at the reaction endpoint

FIG. 1

**Description**

TECHNICAL FIELD

The present application relates to the technical field of hydrometallurgy, and specifically relates to a preparation method for a nickel-cobalt-manganese raw material with ion co-equilibrium in hydrometallurgy of laterite nickel ore.

BACKGROUND

In the context of the new energy era, laterite nickel ore as a key nickel resource has attracted wide attention. Compared with pyrometallurgical technology, laterite nickel ore hydrometallurgical technology has obvious advantages in terms of energy consumption, raw material adaptability, cost, and environmental protection. In the field of hydrometallurgy, the design of the whole process hydrometallurgy is closely related to the balance relationship of various ions. The whole process hydrometallurgy based on laterite nickel ore involves process sections such as high-pressure leaching, cycle leaching, and neutralization, iron and aluminum removal, and MHP (nickel and cobalt hydroxide) precipitation. How to design, evaluate, and optimize the process conditions of each process section depends on the balance relationship of various ions in the aqueous solution.

Currently, the MHP products prepared by the hydrometallurgical process of laterite nickel ore have uneven quality. The MHP precipitation as the process section of product output in the whole process hydrometallurgy of laterite nickel ore has a very important influence on the product. In the process of MHP precipitation of laterite nickel ore, the type of precipitating agent, the additive amount of precipitating agent, and the reaction degree have a decisive impact on the quality of MHP. In actual industrial production, the additive amount of precipitating agent and the reaction degree are generally evaluated artificially through experience. However, if the composition of the nickel-cobalt solution changes, it is impossible to accurately determine the additive amount of precipitating agent and the reaction degree, which ultimately affects the quality of MHP products.

SUMMARY

An object of the present application is to overcome the above technical shortcomings, and provides a preparation method for a nickel-cobalt-manganese raw material with ion co-equilibrium in hydrometallurgy of laterite nickel ore, which solves the technical problem of uneven quality of MHP products caused by artificial evaluation on the reaction degree through experience in the prior art.

The present application provides a preparation method for a nickel-cobalt-manganese raw material with ion co-equilibrium in hydrometallurgy of laterite nickel ore, which includes the following steps:

acquiring parameters of an initial material liquid, an actual pH of a material liquid at a reaction endpoint, and a target precipitation rate of a target metal;

establishing a first theoretical relationship model between a theoretical pH of the material liquid at the reaction endpoint and the target precipitation rate of the target metal, and obtaining the theoretical pH of the material liquid at the reaction endpoint based on the parameters of the initial material liquid and the first theoretical relationship model; and

judging whether to terminate the reaction based on the theoretical pH and the actual pH of the material liquid at the reaction endpoint.

Compared with the prior art, the present application includes the following beneficial effects.

In the present application, a first theoretical relationship model between the theoretical pH of the material liquid at the reaction endpoint and the target precipitation rate of the target metal is established from the perspective of ion equilibrium, guiding the MHP precipitation process. Through the method of the present application, the theoretical pH of the material liquid at the reaction endpoint can be directly obtained based on the parameters of the initial material liquid and the target precipitation rate of the target metal, thus judging whether to terminate the reaction through the theoretical pH of the material liquid at the reaction endpoint and the actual pH of the material liquid at the reaction endpoint; simultaneously, the test method of the actual pH of the material liquid at the reaction endpoint is simpler and more intuitive than that of the metal concentration in the material liquid at the reaction endpoint, which is easier to directly judge the reaction degree by pH to regulate the reaction process.

BRIEF DESCRIPTION OF DRAWINGS

FIG. 1 shows a process flow chart of an embodiment of the preparation method for a nickel-cobalt-manganese raw material with ion co-equilibrium in hydrometallurgy of laterite nickel ore provided in the present application; and

FIG. 2 shows a relationship diagram between the target precipitation rates of nickel, cobalt, manganese, and magnesium and the theoretical pH of the material liquid at the reaction endpoint established based on the first theoretical relationship model in Embodiment 1 of the present application.

DETAILED DESCRIPTION

In order to make the purpose, technical solution, and advantages of the present application more clearly, the present application is further described in detail below in conjunction with the drawings and embodiments. It should be understood that the embodiments described herein are merely used for illustrating the present application and should not be regarded as a specific limitation to the present application.

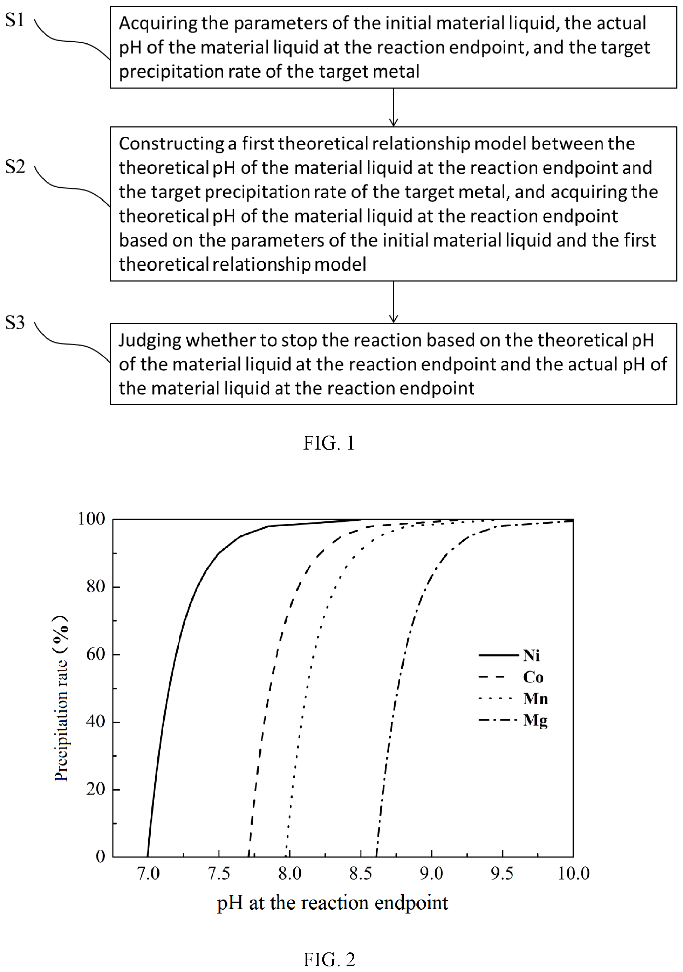

Referring to FIG. 1, the present application provides a preparation method for a nickel-cobalt-manganese raw material with ion co-equilibrium in hydrometallurgy of laterite nickel ore, which includes the following steps:

S1: acquiring parameters of an initial material liquid, an actual pH of the material liquid at a reaction endpoint, and a target precipitation rate of a target metal; wherein the parameters of the initial material liquid include components and volume of the initial material liquid;

S2: establishing a first theoretical relationship model between a theoretical pH of the material liquid at the reaction endpoint and the target precipitation rate of the target metal, and obtaining the theoretical pH of the material liquid at the reaction endpoint based on the parameters of the initial material liquid and the first theoretical relationship model; and

S3: judging whether to terminate the reaction based on the theoretical pH of the material liquid at the reaction endpoint and the actual pH of the material liquid at the reaction endpoint.

In the present application, a first theoretical relationship model between the theoretical pH of the material liquid at the reaction endpoint and the target precipitation rate of the target metal is established from the perspective of ion equilibrium, guiding the MHP precipitation process. Through the method of the present application, the theoretical pH of the material liquid at the reaction endpoint can be directly obtained based on the parameters of the initial material liquid and the target precipitation rate of the target metal, thus judging whether to terminate the reaction through the theoretical pH of the material liquid at the reaction endpoint and the actual pH of the material liquid at the reaction endpoint; simultaneously, the test method of the actual pH of the material liquid at the reaction endpoint is simpler and more intuitive than that of the metal concentration in the material liquid at the reaction endpoint, which is easier to directly judge the reaction degree by pH to regulate the reaction process.

In the present application, the source of the initial material liquid is not limited, and those skilled in the art can choose according to the actual situation. In some embodiments of the present application, the initial material liquid is a solution after removal of iron and aluminum of laterite nickel ore, which can react with a precipitating agent to prepare a MHP slurry.

Since the key target metal element for the recovery of laterite nickel ore hydrometallurgy is nickel, and after the determination of the precipitation rate of nickel, the precipitation rate of cobalt and manganese, and the component of MHP can be basically determined. Therefore, in some embodiments of the present application, the target metal is nickel.

In the present application, the type of precipitating agent is not limited, and those skilled in the art can choose according to the actual situation. In some embodiments of the present application, the precipitating agent may be a sodium hydroxide solution and/or a magnesium oxide slurry.

In the embodiment, the initial material liquid and the precipitating agent are subjected to a reaction to obtain a slurry, the slurry is subjected to solid-liquid separation to obtain a filtrate, and the filtrate is detected to obtain the actual pH of the material liquid at the reaction endpoint. In the present application, the manner of the solid-liquid separation is not limited, and those skilled in the art can choose according to the actual situation. For embodiment, the solid-liquid separation can be achieved through filtration.

In some embodiments of the present application, the material liquid at the reaction endpoint is obtained by the following method:

mixing the initial material liquid and the precipitating agent and performing a reaction, to obtain a MHP slurry;

performing a thickening treatment on the MHP slurry to obtain a MHP dense underflow; and

filtering the MHP dense underflow to obtain a MHP filter cake and a filtrate, and the filtrate is the material liquid at the reaction endpoint.

Further, the MHP filter cake is washed with water to obtain washed water, and the actual precipitation rate is calculated after sampling and detecting the filtrate and washed water.

In the embodiment, the calculation formula corresponding to the first theoretical relational model in step S2 is as follows:

$$R = (1 - \frac{K_{sp} \times A}{C_i (K_w \times 10^{pH})^X}) \times 100\% \qquad (1)$$

wherein $K_{sp}$ refers to solubility product constant, $K_w$ refers to the ion product of water, $C_i$ refers to the mass concentration of the target metal in the initial material liquid, A refers to the relative atomic mass of the target metal, R refers to the target precipitation rate of the target metal, pH refers to the theoretical pH of the material liquid at the reaction endpoint, and X refers to the chemical valence state of the target metal.

Specifically, in step S2, the step of establishing a first theoretical relationship model between a theoretical pH of the material liquid at the reaction endpoint and the target precipitation rate of the target metal includes:

S21: establishing a model of the solubility product constant and a model of the ion product of water;

wherein the model of solubility product constant is established based on the hydrolytic equilibrium reaction equation of the target metal, and the hydrolytic equilibrium reaction equation of the target metal is:

$$M^{X+} + XOH^- \rightleftarrows M(OH)_X$$

wherein X refers to the chemical valence state of the target metal.

The solubility product constant ($K_{sp}$), abbreviated as the solubility product, represents the product of the power of the concentrations of each ion when the precipitate reaches the precipitate-dissolution equilibrium state in the solution. The concentration of the target metal in the material liquid at the reaction endpoint and the solubility product constant ($K_{sp}$) when the target metal reaches the precipitate-dissolution equilibrium satisfy the following model of the solubility product constant:

$$Ksp = \frac{C_f}{A} \times \left[ C_f(OH)^- \right]^X \qquad (2)$$

wherein A refers to the relative atomic mass of the target metal, $C_f$ refers to the mass concentration of the target metal in the material liquid at the reaction endpoint, $C_f(OH^-)$ refers to the molar concentration of the $OH^-$ ion in the material liquid at the reaction endpoint, $K_{sp}$ is only related to temperature or pressure; at atmospheric pressure and temperature of 25°C, the $K_{sp}$ of $Ni(OH)_2$ is $5.92 \times 10^{-16}$, the $K_{sp}$ of $Co(OH)_2$ is $1.6 \times 10^{-15}$, the $K_{sp}$ of $Mn(OH)_2$ is $4 \times 10^{-14}$, and the $K_{sp}$ of $Mg(OH)_2$ is $5.61 \times 10^{-12}$,

The ion product constant of water, abbreviated as the ion product of water, represents the product of the concentration of ionization product $H^+$ and the concentration of $OH^-$ when water reaches ionization equilibrium, and the concentration of the $OH^-$ ion in the material liquid at the reaction endpoint and the theoretical pH of the material liquid at the reaction endpoint satisfy the following model of the ion product constant of water:

$$K_w = 10^{-pH} \times C_f(OH^-) \qquad (3)$$

wherein, the ion product constant of water $K_w$ only related to temperature or pressure; at atmospheric pressure and temperature of 25°C, $K_w$ is $1 \times 10^{-14}$, and pH refers to the theoretical pH of the material liquid at the reaction endpoint.

S22: establishing a theoretical relationship model between the precipitation rate of the target metal and parameters of the initial material liquid and parameters of the material liquid at the reaction endpoint;

wherein a calculation formula corresponding to the theoretical relationship model between the precipitation rate of the target metal and parameters of the initial material liquid and parameters of the material liquid at the reaction endpoint:

$$R = \frac{C_i V_i - C_f V_f}{C_i V_i} \times 100\% \qquad (4)$$

wherein R refers to the target precipitation rate of the target metal; $C_i$ and $C_f$ refer to the mass concentrations of the metal before and after the hydrolysis reaction, respectively (i.e., the mass concentration of the target metal in the initial material liquid and the mass concentration of the target metal in the material liquid at the reaction endpoint, respectively); $V_i$ and $V_f$ refer to the volumes of the solution before and after the hydrolysis reaction, respectively (i.e., the volume of the initial material liquid and the volume of the material liquid at the reaction endpoint, respectively); due to the relatively small volume of the precipitating agent added compared to the initial material liquid during the MHP precipitation process, it can be considered that $V_i \approx V_f$.

S23: establishing the first theoretical relationship model between the theoretical pH of the material liquid at the reaction endpoint and the target precipitation rate of the target metal based on the model of solubility product constant, the model of the ion product constant of water, and the theoretical relationship model between the precipitation rate of the target metal and parameters of the initial material liquid and parameters of the material liquid at the reaction endpoint.

Step S23 specifically includes: combining (2), (3), and (4), to obtain the first theoretical relationship model between the theoretical pH of the material liquid at the reaction endpoint and the target precipitation rate of the target metal.
In the embodiment, the following step is further included before step S2: establishing a second theoretical relationship model between a precipitating agent dosage and the target precipitation rate of the target metal, and the precipitating agent dosage is obtained based on parameters of the initial material liquid and the second theoretical relationship model.
Specifically, the calculation formula corresponding to the second theoretical relationship model is as follows:

$$m = \left(\frac{C_{Ni}}{58.69} + \frac{C_{Co}}{58.93}\right)\left(\frac{R \times B \times \eta}{P}\right) \qquad (5)$$

wherein m refers to the precipitating agent dosage; $C_{Ni}$ refers to the mass concentration of nickel ion in the initial material liquid; $C_{Co}$ refer to the mass concentration of cobalt ion in the initial material liquid; R refers to the target precipitation rate of the target metal; B refers to the molecular mass of precipitating agent; P refers to the mass concentration of precipitating agent; $\eta$ refers to the coefficient of precipitating agent dosage obtained by fitting experimental results.
Specifically, the precipitating agent is a sodium hydroxide solution, and the coefficient $\eta$ of precipitating agent dosage is 2.08; or, the precipitating agent is a magnesium oxide slurry, and the coefficient $\eta$ of precipitating agent dosage is 0.95.
In the method of the present application, a second theoretical relationship model between the precipitating agent dosage and the target precipitation rate of the target metal is established, guiding the MHP precipitation process. Through the method of the present application, the precipitating agent dosage can be directly obtained based on the parameters of the initial material liquid and the target precipitation rate of the target metal.
In the embodiment, the step of judging whether to terminate the reaction based on the theoretical pH of the material liquid at the reaction endpoint and the actual pH of the material liquid at the reaction endpoint in step S3 includes: terminating the reaction if a ratio of the actual pH to the theoretical pH of the material liquid at the reaction endpoint is between 99% and 101%.
In the method of the present application, if the ratio of the actual pH of the material liquid at the reaction endpoint to the theoretical pH of the material liquid at the reaction endpoint is less than 99, that is, if the reaction does not reach the target reaction degree, the reaction is continued until the ratio of the actual pH of the material liquid at the reaction endpoint to the theoretical pH of the material liquid at the reaction endpoint reaches 99-101%, and the reaction is terminated.
In the present application, taking the currently industrialized precipitating agent sodium hydroxide solution and magnesium oxide slurry as embodiments, and from the perspective of ion equilibrium, the first theoretical relationship model between the theoretical pH of the material liquid at the reaction endpoint and the target precipitation rate of the target metal and the second theoretical relationship model between the precipitating agent dosage during the MHP precipitation process and the target precipitation rate of the target metal are established, guiding the MHP precipitation process. Based on the theory of ion equilibrium and practical experience, it is found that in a case where the precipitating agent is the sodium hydroxide solution, the conditions for the theory to be established are: the target precipitation rate of nickel is less than or equal to 93%, a mass concentration of sodium hydroxide solution is 5-15%, a reaction time is $0.5\,h \leq t \leq 3\,h$, and a reaction temperature is $55°C \leq T \leq 70°C$; in a case where the precipitating agent is the magnesium oxide slurry, the conditions for the theory to be established are: the target precipitation rate of nickel is less than or equal to 98%, a mass concentration of magnesium oxide slurry is 3-15%, a reaction time is $4\,h \leq t \leq 8\,h$, and a reaction temperature is $55°C \leq T \leq 70°C$. The methods proposed in the present application are all valid within the above scope. In addition, MHP precipitation is generally divided into two stages at the current time. In order to ensure the quality of output MHP products and reduce the cost of the subsequent extraction section, the first stage of the MHP precipitation section is the product output grade with a precipitation rate of nickel of less than or equal to 85% in general. Simultaneously, the precipitation rate of nickel in the product out section of MHP precipitation is about 80% in the actual production, thereby the method of the present application is sufficient to guide the actual production.

In Embodiments and Comparative Embodiments of the present application, Embodiment 1 is used to illustrate the relationship between the theoretical pH of the material liquid at the reaction endpoint and the target precipitation rate of the target metal, and Embodiments 2-5 and Comparative Embodiments 1-3 are combined with specific MHP precipitation process to further verify the above method.

In order to avoid repetition, the composition of the nickel-cobalt solution used in the following Embodiments and Comparative Embodiments in the present application is shown in Table 1.

Table 1 Components of nickel-cobalt solution of iron-aluminium-removed laterite nickel ore used for preparing MHP (g/L)

| Ni | Co | Mn | Mg |
|---|---|---|---|
| 3.52 | 0.36 | 2.52 | 8.2 |

**Embodiment 1**

According to the first theoretical model proposed in the present application, it can be inferred that after the MHP reaction completed, the theoretical pH of the material liquid at the reaction endpoint is not related to the type of precipitating agent, but only to the target precipitation rate of the target metal, and the target precipitation rate of the target metal and the theoretical pH of the material liquid at the reaction endpoint meet the relationship of Equation (1).

Nickel was taken as an example; if the target precipitation rates of nickel are 80%, 90%, and 95%, the theoretical pH of the material liquid at the reaction endpoint can be calculated by bringing each parameter into Equation (1), respectively. Further, the target precipitation rate of nickel of 80% was taken as an example, and the relationship between the target precipitation rate of nickel and the theoretical pH of the material liquid at the reaction endpoint was as follows:

$$80\% = (1 - \frac{5.92 \times 10^{-16} \times 58.69}{3.52(1 \times 10^{-14} \times 10^{pH})^2}) \times 100\%$$

and pH at the endpoint = 7.35 was obtained. In Equation (1), $K_w$ refers to $1 \times 10^{-14}$, and $K_{sp}$ refers to $5.92 \times 10^{-16}$.

In the same way, if the target precipitation rate of nickel was 90% and 95%, respectively, the theoretical pH of the material liquid at the reaction endpoint was 7.50 and 7.65, respectively.

The equilibrium constant of the hydrolysis and precipitation reaction $K_{sp}$ ($K_{sp}$ was $5.92 \times 10^{-16}$, $1.6 \times 10^{-15}$, $4 \times 10^{-14}$, $5.61 \times 10^{-12}$, respectively) of valuable metal ions nickel, cobalt, manganese, and magnesium in nickel-cobalt solution was brought into the first theoretical model proposed in the present application, respectively, and the relationship between the target precipitation rates of nickel, cobalt, manganese, and magnesium and the theoretical pH of the material liquid at the reaction endpoint during the MHP precipitation reaction can be obtained, as shown in FIG. 2.

Referring to FIG. 2, it can be seen that the hydrolysis sequence of nickel, cobalt, manganese, and magnesium is nickel > cobalt > manganese > magnesium; in a case where the theoretical pH of the material liquid at the reaction endpoint is less than 8 and under the same pH conditions, magnesium will basically not be hydrolyzed, and manganese has little influence on the hydrolysis of nickel and cobalt, thus the dosage of alkali in the preparation of MHP can be calculated according to the concentration of nickel and cobalt in nickel-cobalt solution, which is the theoretical basis for the establishing of the second theoretical model.

The hydrometallurgy of laterite nickel ore is mainly based on recovering nickel, followed by cobalt. Therefore, in the present application, the first theoretical model and the second theoretical model proposed in the present application are verified emphatically from the actual precipitation rate of nickel and the pH of the actual reaction endpoint.

**Embodiment 2**

(1) 48% sodium hydroxide solution was mixed with industrial water to obtain an alkaline solution with a mass concentration of 5%.

(2) A solution after removal of iron and aluminum of laterite nickel ore was mixed with the alkaline solution, and the mixture was subjected to a precipitation reaction to obtain an MHP slurry under a reaction temperature of 65°C for a reaction time of 2 h. In this process section, the target precipitation rate of nickel needed to be controlled at 80%, and the mass concentration of nickel and cobalt in Table 1 was brought into Equation (5), and the additive amount m of 48% sodium hydroxide solution was obtained to be:

$$m = (\frac{3.52}{58.69} + \frac{0.36}{58.93})(\frac{80\% \times 40 \times 2.08}{48\%})$$

that is, the additive mass of 48% sodium hydroxide solution per liter of nickel-cobalt solution is m = 9.16 g. In Equation (5), the molecular mass of sodium hydroxide was 40 g/mol and $\eta$ was 2.08.

(3) The MHP slurry was subjected to a thickening treatment to obtain an MHP dense underflow.

(4) The MHP dense underflow was subjected to filtration to obtain an MHP filter cake and filtrate; the filtrate was sampled and tested, and the actual pH of the material liquid at the reaction endpoint was found to be 7.38, which was basically consistent with the theoretical pH of the material liquid at the reaction endpoint. In addition, the MHP filter cake was washed with industrial water to obtain washed water, the filtrate and the washed water were sampled and tested, and the actual precipitation rate of nickel was calculated to be 80.79%, which was also very close to the target precipitation rate of nickel, verifying the rationality of the method in the present application.

(5) Part of the thickened underflow was returned to the MHP precipitation process section as crystal seeds.

**Embodiment 3**

This embodiment differs from Embodiment 2 in that: 48% sodium hydroxide solution was mixed with industrial water to obtain an alkaline solution with a mass fraction of 10%, and a precipitation reaction was performed; the actual precipitation rate of nickel was calculated to be 79.16%, and the actual pH of the material liquid at the reaction endpoint was 7.31. The increase in the concentration of sodium hydroxide solution will lead to local overalkalinity, thereby the precipitation rate of nickel slightly decreased. However, within the concentration range of the alkaline solution commonly used in production and specified in the present application, the relationship between the dosage of sodium hydroxide and the precipitation rate of nickel proposed in the present application is applicable.

**Embodiment 4**

This embodiment differs from Embodiment 2 in that: the target precipitation rate of nickel was 90%.
The additive amount of 48% sodium hydroxide solution m was calculated to be:

$$m = (\frac{3.52}{58.69} + \frac{0.36}{58.93})(\frac{90\% \times 40 \times 2.08}{48\%})$$

that is, the additive mass of 48% sodium hydroxide solution per liter of nickel-cobalt solution is m = 10.31 g.
The filtrate was sampled and tested, and the actual pH of the material liquid at the reaction endpoint was found to be 7.45, and the actual precipitation rate of nickel was calculated to be 88.25%. It can be found that there was a difference of 1.75% between the actual precipitation rate and the target precipitation rate, but it has no effect on the quality of MHP products. However, with the increasing target precipitation rate of nickel, the actual precipitation rate of nickel under the calculated dosage of sodium hydroxide condition will deviate more from the target precipitation rate; it was found by tests that the relationship between the dosage of sodium hydroxide and the precipitation rate of nickel proposed in the present application is used as far as possible under the target precipitation rate of nickel is less than or equal to 93%.

**Embodiment 5**

In this embodiment, the composition of the nickel-cobalt solution was the same as that of Embodiment 2, and this embodiment differs from Embodiment 2 in that: magnesium oxide was used as the precipitating agent.

(1) Magnesium oxide with a mass fraction of 93% was mixed with industrial water to obtain an alkaline slurry with a mass concentration of 5%.
(2) A solution after removal of iron and aluminum of laterite nickel ore was mixed with the alkaline slurry, and the mixture was subjected to a precipitation reaction to obtain an MHP slurry under a reaction temperature of 65°C for a reaction time of 5 h. If the target precipitation rate of nickel needed to be controlled at 80% in this process section, the mass concentration of nickel and cobalt in Table 1 was brought into Equation (5), and the additive amount m of 93% magnesium oxide was obtained to be:

$$m = (\frac{3.52}{58.69} + \frac{0.36}{58.93})(\frac{80\% \times 40.3 \times 0.95}{93\%})$$

that is, the additive mass of 93% magnesium oxide per liter of nickel-cobalt solution is m = 2.17 g. In Equation (5), the molecular mass of magnesium oxide was 40.3 g/mol and $\eta$ was 0.95;
(3) The MHP slurry was subjected to a thickening treatment to obtain an MHP dense underflow.
(4) The dense underflow was subjected to filtration to obtain an MHP filter cake and filtrate; the filtrate was sampled and tested, and the actual pH of the material liquid at the reaction endpoint was found to be 7.40, which was basically consistent with the theoretical pH of the material liquid at the reaction endpoint; in addition, the MHP was washed with industrial water to obtain washed water, the filtrate and the washed water were sampled and tested, and the actual precipitation rate of nickel was calculated to be 80.88%, which was also very close to the target precipitation rate of nickel, indicating that the method in the present application can also be well applied to the system with magnesium oxide as the precipitating agent.

**Embodiment 6**

This embodiment differs from Embodiment 5 in that: the target precipitation rate of nickel was 95%. The additive amount of 93% magnesium oxide m was calculated to be:

$$m = (\frac{3.52}{58.69} + \frac{0.36}{58.93})(\frac{95\% \times 40.3 \times 0.95}{93\%})$$

that is, the additive mass of 93% magnesium oxide per liter of nickel-cobalt solution is m = 2.58 g. It was found that the actual precipitation rate of nickel was tested to be 95.45%, and the actual pH of the material liquid at the reaction endpoint equaled to 7.70.

**Comparative Embodiment 1**

This embodiment differs from Embodiment 2 in that: the MHP precipitation reaction was performed for a period of 0.2 h, and other reaction conditions were the same.
After testing, it was found that the actual pH of the material liquid at the reaction endpoint was 7.48, and the actual precipitation rate of nickel was calculated to be 83.61%. It can be found that there was a large difference between the actual reaction endpoint pH = 7.48 and the theoretical endpoint pH of 7.35 calculated by Equation (1). Simultaneously, although nickel is easier to hydrolyze than cobalt and manganese, and the nickel precipitation rate is high at a reaction time of 0.2 h, the MHP slurry with a small particle size is difficult to filter-press due to the short reaction time.

**Comparative Embodiment 2**

This embodiment differs from Embodiment 2 in that: the MHP precipitation reaction was performed for a period of 4 h, and other reaction conditions were the same.
After testing, it was found that the actual pH of the material liquid at the reaction endpoint was 7.26 (theoretical pH at the reaction endpoint = 7.35), and the actual precipitation rate of nickel was only 75.25%. Therefore, in a case where sodium hydroxide is used as the precipitating agent, the reaction time should not be too long; a too long the reaction time will promote the oxidation and hydrolysis reaction of manganese and lead to a decrease in the precipitation rate of nickel.

**Comparative Embodiment 3**

This embodiment differs from Embodiment 5 in that: the MHP precipitation reaction was performed for a period of 1.5 h, and other reaction conditions were the same.

After testing, it was found that the actual pH of the material liquid at the reaction endpoint was 7.24 (theoretical pH at the reaction endpoint = 7.35), and the precipitation rate of nickel was only 73.46%. Therefore, in a case where magnesium oxide is used as the precipitating agent, a too short reaction time will lead to a low precipitation rate of nickel.

**Comparative Embodiment 4**

This embodiment differs from Embodiment 5 in that: the MHP precipitation reaction was performed for a period of 9 h, and other reaction conditions were the same.

After testing, it was found that the actual pH of the material liquid at the reaction endpoint was 7.43 (theoretical pH at the reaction endpoint = 7.35), and the precipitation rate of nickel was 82.93%. In a case where magnesium oxide is used as the precipitating agent, the precipitation rate of nickel will increase due to a too long reaction time, but the precipitation rate of cobalt will decrease to 66.83%. In order to reduce the cost of transportation, an appropriate reaction time is needed.

The specific embodiments of the present application described above do not constitute a limitation of the protection scope of the present application. Any other consequential alterations and deformations made in accordance with the technical conception of the present application shall be fall within the protection scope of the claims of the present application.

**Claims**

1. A preparation method for a nickel-cobalt-manganese raw material with ion co-equilibrium in hydrometallurgy of laterite nickel ore, comprising the following steps:

   acquiring parameters of an initial material liquid, an actual pH of a material liquid at a reaction endpoint, and a target precipitation rate of a target metal;
   establishing a first theoretical relationship model between a theoretical pH of the material liquid at the reaction endpoint and the target precipitation rate of the target metal, and obtaining the theoretical pH of the material liquid at the reaction endpoint based on the parameters of the initial material liquid and the first theoretical relationship model; and
   judging whether to terminate the reaction based on the theoretical pH and the actual pH of the material liquid at the reaction endpoint.

2. The preparation method for a nickel-cobalt-manganese raw material with ion co-equilibrium in hydrometallurgy of laterite nickel ore according to claim 1, wherein the parameters of the initial material liquid are obtained by detecting a solution after removal of iron and aluminum from laterite nickel ore; the actual pH of the material liquid at the reaction endpoint is obtained by detecting the filtrate obtained from solid-liquid separation of the slurry after the reaction between the initial material liquid and a precipitating agent.

3. The preparation method for a nickel-cobalt-manganese raw material with ion co-equilibrium in hydrometallurgy of laterite nickel ore according to claim 1, wherein a calculation formula corresponding to the first theoretical relational model is as follows:

$$R = \left(1 - \frac{K_{sp} \times A}{C_i(K_w \times 10^{pH})^X}\right) \times 100\% \qquad (1)$$

wherein $K_{sp}$ refers to solubility product constant, $K_w$ refers to the ion product of water, $C_i$ refers to a mass concentration of the target metal in the initial material liquid, A refers to a relative atomic mass of the target metal, R refers to a target precipitation rate of the target metal, pH refers to a theoretical pH of the material liquid at the reaction endpoint, and X refers to a chemical valence state of the target metal.

4. The preparation method for a nickel-cobalt-manganese raw material with ion co-equilibrium in hydrometallurgy of laterite nickel ore according to claim 1, wherein the step of establishing a first theoretical relationship model between a theoretical pH of the material liquid at the reaction endpoint and the target precipitation rate of the target metal comprises:

establishing a model of the solubility product constant and a model of the ion product of water;
establishing a theoretical relationship model between the precipitation rate of the target metal and parameters of the initial material liquid and parameters of the material liquid at the reaction endpoint; and
establishing the first theoretical relationship model between the theoretical pH of the material liquid at the reaction endpoint and the target precipitation rate of the target metal based on the model of solubility product constant, the model of the ion product constant of water, and the theoretical relationship model between the precipitation rate of the target metal and parameters of the initial material liquid and parameters of the material liquid at the reaction endpoint.

5. The preparation method for a nickel-cobalt-manganese raw material with ion co-equilibrium in hydrometallurgy of laterite nickel ore according to claim 4, wherein a calculation formula corresponding to the solubility product constant model is as follows:

$$Ksp = \frac{C_f}{A} \times \left[ C_f(OH)^- \right]^X \qquad (2)$$

wherein A refers to a relative atomic mass of the target metal, $C_f$ refers to a mass concentration of the target metal in the material liquid at the reaction endpoint, $C_f(OH^-)$ refers to a molar concentration of the $OH^-$ ion in the material liquid at the reaction endpoint;
a calculation formula corresponding to the ion product constant model is as follows:

$$K_w = 10^{-pH} \times C_f(OH^-) \qquad (3)$$

wherein pH refers to a theoretical pH of the material liquid at the reaction endpoint;
a calculation formula corresponding to the theoretical relationship model between the precipitation rate of the target metal and parameters of the initial material liquid and parameters of the material liquid at the reaction endpoint is as follows:

$$R = \frac{C_i V_i - C_f V_f}{C_i V_i} \times 100\% \qquad (4)$$

wherein R refers to a target precipitation rate of the target metal; $C_i$ and $C_f$ refer to a mass concentration of the target metal in the initial material liquid and a mass concentration of the target metal in the material liquid at the reaction endpoint, respectively; $V_i$ and $V_f$ refer to an volume of the initial material liquid and an volume of the material liquid at the reaction endpoint, respectively, and $Vi \approx Vf$.

6. The preparation method for a nickel-cobalt-manganese raw material with ion co-equilibrium in hydrometallurgy of laterite nickel ore according to claim 1, wherein prior to establishing the first theoretical relationship model between the theoretical pH of the material liquid at the reaction endpoint and the target precipitation rate of the target metal, the method further comprises:
establishing a second theoretical relationship model between a precipitating agent dosage and the target precipitation rate of the target metal, and the precipitating agent dosage is obtained based on parameters of the initial material liquid and the second theoretical relationship model.

7. The preparation method for a nickel-cobalt-manganese raw material with ion co-equilibrium in hydrometallurgy of laterite nickel ore according to claim 6, wherein a calculation formula corresponding to the second theoretical relationship model is as follows:

$$m = \left(\frac{C_{Ni}}{58.69} + \frac{C_{Co}}{58.93}\right)\left(\frac{R \times B \times \eta}{P}\right) \qquad (5)$$

wherein m refers to a precipitating agent dosage; $C_{Ni}$ refers to a mass concentration of nickel ion in the initial material liquid; $C_{Co}$ refer to a mass concentration of cobalt ion in the initial material liquid; R refers to a target precipitation rate of the target metal; B refers to a molecular mass of precipitating agent; P refers to a mass concentration of precipitating agent; η refers to a coefficient of precipitating agent dosage obtained by fitting experimental results.

8. The preparation method for a nickel-cobalt-manganese raw material with ion co-equilibrium in hydrometallurgy of laterite nickel ore according to claim 7, wherein the target metal is nickel, the precipitating agent is a sodium hydroxide solution, and the coefficient of precipitating agent dosage is 2.08; or, the target metal is nickel, the precipitating agent is an magnesium oxide slurry, and the coefficient of precipitating agent dosage is 0.95.

9. The preparation method for a nickel-cobalt-manganese raw material with ion co-equilibrium in hydrometallurgy of laterite nickel ore according to claim 1, wherein the target metal is nickel, the precipitating agent is the sodium hydroxide solution, a target precipitation rate of the target metal is less than or equal to 93%, a mass concentration of sodium hydroxide solution is 5-15%, a reaction time is $0.5\,h \leq t \leq 3\,h$, and a reaction temperature is $55°C \leq T \leq 70°C$; or, the target metal is nickel, the precipitating agent is the magnesium oxide slurry, the target precipitation rate of the target metal is less than or equal to 98%, a mass concentration of magnesium oxide slurry is 3-15%, a reaction time is $4\,h \leq t \leq 8\,h$, and a reaction temperature is $55°C \leq T \leq 70°C$.

10. The preparation method for a nickel-cobalt-manganese raw material with ion co-equilibrium in hydrometallurgy of laterite nickel ore according to claim 1, wherein the step of judging whether to terminate the reaction based on the theoretical pH and the actual pH of the material liquid at the reaction endpoint comprises: terminating the reaction if a ratio of the actual pH to the theoretical pH of the material liquid at the reaction endpoint is between 99% and 101%.

S1 — Acquiring the parameters of the initial material liquid, the actual pH of the material liquid at the reaction endpoint, and the target precipitation rate of the target metal

S2 — Constructing a first theoretical relationship model between the theoretical pH of the material liquid at the reaction endpoint and the target precipitation rate of the target metal, and acquiring the theoretical pH of the material liquid at the reaction endpoint based on the parameters of the initial material liquid and the first theoretical relationship model

S3 — Judging whether to stop the reaction based on the theoretical pH of the material liquid at the reaction endpoint and the actual pH of the material liquid at the reaction endpoint

FIG. 1

FIG. 2

## INTERNATIONAL SEARCH REPORT

| International application No. |
|---|
| **PCT/CN2024/122777** |

**A. CLASSIFICATION OF SUBJECT MATTER**

C22B23/00(2006.01)i

According to International Patent Classification (IPC) or to both national classification and IPC

**B. FIELDS SEARCHED**

Minimum documentation searched (classification system followed by classification symbols)

IPC:C22B

Documentation searched other than minimum documentation to the extent that such documents are included in the fields searched

Electronic data base consulted during the international search (name of data base and, where practicable, search terms used)

CNTXT, ENTXTC, VEN, CNKI, WEB OF SCIENCE: 红土镍矿, 镍, 钴, 锰, 沉淀, pH, 碱, 氢氧化钠, 氧化镁, 理论, 模型, nickel, cobalt, manganese, laterite, precipitation, theoretical, sodium hydroxide, magnesium oxide, model, NaOH, MgO, MHP

**C. DOCUMENTS CONSIDERED TO BE RELEVANT**

| Category* | Citation of document, with indication, where appropriate, of the relevant passages | Relevant to claim No. |
|---|---|---|
| X | 周正恩 (ZHOU, Zhengen). "褐铁型红土镍矿硝酸加压浸出过程有价组元的富集与提取技术基础 (Extraction of Valuable Components during Nitric Acid Pressure Leaching Process of Treating Limonitic Laterite)" 中国博士论文全文数据库 工程科技I辑 (Engineering Science & Technology I, China Doctoral Dissertations Full-Text Database), 15 February 2024 (2024-02-15), sections 4.1-4.3 | 1-5, 9-10 |
| Y | 周正恩 (ZHOU, Zhengen). "褐铁型红土镍矿硝酸加压浸出过程有价组元的富集与提取技术基础 (Extraction of Valuable Components during Nitric Acid Pressure Leaching Process of Treating Limonitic Laterite)" 中国博士论文全文数据库 工程科技I辑 (Engineering Science & Technology I, China Doctoral Dissertations Full-Text Database), 15 February 2024 (2024-02-15), sections 4.1-4.3 | 6-8 |
| Y | US 2002031463 A1 (CENTAUR NICKEL PTY LTD.) 14 March 2002 (2002-03-14) the claims | 6-8 |
| A | CN 117280054 A (QINGMEIBANG NEW ENERGY MATERIALS CO., LTD. et al.) 22 December 2023 (2023-12-22) entire document | 1-10 |

☑ Further documents are listed in the continuation of Box C.   ☑ See patent family annex.

| | |
|---|---|
| * Special categories of cited documents: <br> "A" document defining the general state of the art which is not considered to be of particular relevance <br> "D" document cited by the applicant in the international application <br> "E" earlier application or patent but published on or after the international filing date <br> "L" document which may throw doubts on priority claim(s) or which is cited to establish the publication date of another citation or other special reason (as specified) <br> "O" document referring to an oral disclosure, use, exhibition or other means <br> "P" document published prior to the international filing date but later than the priority date claimed | "T" later document published after the international filing date or priority date and not in conflict with the application but cited to understand the principle or theory underlying the invention <br> "X" document of particular relevance; the claimed invention cannot be considered novel or cannot be considered to involve an inventive step when the document is taken alone <br> "Y" document of particular relevance; the claimed invention cannot be considered to involve an inventive step when the document is combined with one or more other such documents, such combination being obvious to a person skilled in the art <br> "&" document member of the same patent family |

| Date of the actual completion of the international search | Date of mailing of the international search report |
|---|---|
| **29 May 2025** | **14 June 2025** |

| Name and mailing address of the ISA/CN | Authorized officer |
|---|---|
| **China National Intellectual Property Administration (ISA/CN)** <br> **China No. 6, Xitucheng Road, Jimenqiao, Haidian District, Beijing 100088** | |
| | Telephone No. |

Form PCT/ISA/210 (second sheet) (July 2022)

**INTERNATIONAL SEARCH REPORT**

International application No.

**PCT/CN2024/122777**

| C. | DOCUMENTS CONSIDERED TO BE RELEVANT |
|---|---|

| Category* | Citation of document, with indication, where appropriate, of the relevant passages | Relevant to claim No. |
|---|---|---|
| A | CN 109022823 A (UNIVERSITY OF SCIENCE AND TECHNOLOGY BEIJING) 18 December 2018 (2018-12-18)<br>entire document | 1-10 |
| A | JP 2020117794 A (SUMITOMO METAL MINING CO., LTD.) 06 August 2020 (2020-08-06)<br>entire document | 1-10 |

Form PCT/ISA/210 (second sheet) (July 2022)

**INTERNATIONAL SEARCH REPORT**
Information on patent family members

International application No.

**PCT/CN2024/122777**

| Patent document cited in search report | | | Publication date (day/month/year) | Patent family member(s) | | | Publication date (day/month/year) |
|---|---|---|---|---|---|---|---|
| US | 2002031463 | A1 | 14 March 2002 | AU | 701829 | B3 | 04 February 1999 |
| | | | | AP | 200001739 | D0 | 31 March 2000 |
| | | | | ZA | 9806718 | B | 15 March 1999 |
| | | | | US | 6409979 | B1 | 25 June 2002 |
| | | | | AU | 751862 | C | 22 February 1999 |
| | | | | AU | 8426098 | A | 29 August 2002 |
| | | | | ID | 24363 | A | 13 July 2000 |
| | | | | OA | 11283 | A | 30 July 2003 |
| | | | | ZA | 986718 | B | 15 March 1999 |
| | | | | AUPO | 837197 | A0 | 28 August 1997 |
| | | | | FR | 2766842 | A1 | 05 February 1999 |
| | | | | FR | 2766842 | B1 | 16 February 2001 |
| | | | | CU | 23081 | A3 | 17 August 2005 |
| | | | | BR | 9811806 | A | 15 August 2000 |
| | | | | CA | 2295066 | A1 | 11 February 1999 |
| | | | | CA | 2295066 | C | 24 February 2009 |
| | | | | AP | 200001739 | A0 | 31 March 2000 |
| | | | | AP | 2000001739 | A0 | 29 May 2002 |
| | | | | WO | 9906603 | A1 | 11 February 1999 |
| CN | 117280054 | A | 22 December 2023 | WO | 2025020176 | A1 | 30 January 2025 |
| | | | | EP | 4524273 | A1 | 19 March 2025 |
| | | | | AU | 2023446745 | A1 | 13 February 2025 |
| | | | | PH | 12024552899 | A1 | 24 March 2025 |
| CN | 109022823 | A | 18 December 2018 | PH | 12020551383 | A1 | 26 July 2021 |
| | | | | CU | 20200059 | A7 | 07 April 2021 |
| | | | | CU | 24672 | B1 | 08 August 2023 |
| | | | | WO | 2020019918 | A1 | 30 January 2020 |
| | | | | AU | 2019310838 | A1 | 13 August 2020 |
| | | | | AU | 2019310838 | B2 | 10 March 2022 |
| | | | | CN | CN109022823 | B | 02 October 2020 |
| JP | 2020117794 | A | 06 August 2020 | JP | 7200698 | B2 | 10 January 2023 |

Form PCT/ISA/210 (patent family annex) (July 2022)